Europäisches Patentamt

European Patent Office

Office européen des brevets

(11) Publication number : **0 503 763 A1**

# (12) EUROPEAN PATENT APPLICATION

(21) Application number : 92301068.0

(22) Date of filing : 07.02.92

(51) Int. Cl.⁵ : **A61K 7/00, A61K 7/42, A61K 7/48, A61K 7/06**

(30) Priority : 08.02.91 JP 17910/91

(43) Date of publication of application :
16.09.92 Bulletin 92/38

(84) Designated Contracting States :
AT BE CH DE DK ES FR GB GR IT LI LU NL PT SE

(71) Applicant : MAX FACTOR K.K.
12-3, Nishigotanda 2-chome
Shinagawa-Ku, Tokyo-to (JP)

(71) Applicant : Sansho Seiyaku Co., Ltd.
26-7, Oike 2-chome
Onojo-shi Fukuoka-ken (JP)

(72) Inventor : Mitani, Hiroaki
6-7 Aoyama 3-chome, Dazaifu-shi
Fukuoka-ken (JP)
Inventor : Shimoda, Hiroko
2-15, 2-chome, Higashikusatsu
Kusatsu-si, Shiga-ken (JP)
Inventor : Yoshii, Takashi
1897-63, Mikami, Yasu-machi
Yasu-gun, Shiga-ken (JP)
Inventor : Tashiro, Keisuke
192, Izumi, Minakuti-cho
Kouga-gun, Shiga-ken (JP)

(74) Representative : Brooks, Maxim Courtney et al
Procter & Gamble (NTC) Limited Whitley Road
Longbenton
Newcastle-upon-Tyne NE12 9TS (GB)

(54) Cosmetic material.

(57)    A cosmetic material containing a purified liquid obtained from liquid cultivation of myelia of Pleurotus sajorcaju. The purified culture liquid of Pleurotus sajorcaju provides an excellent ultraviolet damage inhibing effect, melanogenesis inhibiting effect and antioxidating effect. Where a cosmetic material containing the liquid is applied to the skin, it may effectively prevent aging of the skin eliminating wrinkles and pigmentation, such aging being promoted by ultraviolet rays. Where it is applied to the scalp, it may effectively prevent formation of dandruff and urtication.

EP 0 503 763 A1

Jouve, 18, rue Saint-Denis, 75001 PARIS

## TECHNICAL FIELD

The present invention relates to a cosmetic material containing a purified liquid of a culture liquid of mycelia of Pleurotus sajorcaju (hereinafter simply referred to as a "purified culture liquid," as the case may be), which may prevent aging of the skin and oxidation of sebum.

## BRIEF EXPLANATION OF DRAWINGS

(see attached).

## BACKGROUND OF THE INVENTION

The skin, being different from other organs, is easily influenced by external conditions, such as the physical factors of sun light, temperature, humidity and others, as well as the chemical factors of detergent and so on. Above all, the effects of ultraviolet rays from sunlight on the skin are serious and are known to cause various symptoms of skin aging such as wrinkles, pigmentation and dry skin. Hitherto, cosmetic materials containing ultraviolet absorbent, moisturizers, cell activating agents, antioxidants and the like have been used for the purpose of preventing such symptoms.

However, the conventional cosmetic materials have not displayed sufficient effect and often have a light sensitivity that causes some harmful side effects on the skin. Therefore, their use involves some problems.

Focusing on the above-mentioned situation, the present inventors repeatedly studied cosmetic materials capable of preventing aging of the skin by protecting it from ultraviolet rays. As a result, they have discovered that a purified culture liquid of Pleurotus sajorcaju has an excellent ultraviolet ray damage inhibiting effect, melanogenesis inhibiting effect, and anti-oxidating effect and is very safe for the skin. After further investigation of the liquid, they have also discovered that when a cosmetic material containing such a purified culture liquid is applied to the skin, it is effective for preventing aging of the skin as caused by ultraviolet rays, such as wrinkles, pigmentation and others. Additionally, when it is applied to the scalp, it is effective for preventing oxidation of sebum to thereby inhibit formation of dandruff and urtication of the scalp. On the basis of these findings, they have achieved the present invention.

## SUMMARY OF THE INVENTION

Specifically, the present invention is a cosmetic material comprising a purified culture liquid of mycelia of Pleurotus sajorcaju. Said cosmetic material may be combined with a dermatogically acceptable carrier, diluent or solvent.

## DETAILED DESCRIPTION

The active ingredient to be used in the present invention is derived from the culture liquid of Pleurotus sajorcaju, which is a water-soluble liquid to be obtained by liquid cultivation of mycelia of Pleurotus sajorcaju, by removing the mycelia from the culture liquid, and by purifying the remaining culture liquid. This may be accomplished by any ordinary purification method. Pleurotus sajorcaju belongs to the Basidiomycetes of India growth, the culture liquid of which has been unknown as a useful cosmetic ingredient. In particular, the ultraviolet damage inhibiting effect, the melanogenesis inhibiting effect and the anti-oxidating effect of it was not clarified at all.

Since the purified culture liquid of the present invention has an excellent ultraviolet damage inhibiting effect and melanogenesis inhibiting effect, a cosmetic material containing it will be effective when applied to the skin for retarding symptoms of aging of the skin, such as wrinkles and pigmentation, to be caused by harmful ultraviolet rays. Additionally, when it is applied to the scalp, it will be effective for preventing formation of dandruff and urtication of the scalp, as it also has an anti-oxidating effect.

The form of the cosmetic material of the present invention is not specifically limited, provided that it may be applied to the skin and to the scalp. For instance, a purified culture liquid of Pleurotus sajorcalu, which is an active ingredient of the cosmetic material of the present invention, may be formulated into a cosmetic lotion, cream, emulsion or hair tonic along with ordinary cosmetic bases, aids and additives by an ordinary formulation method.

The amount of the purified culture liquid to be in the cosmetic material of the present invention may be from 0.01 to 50% by weight, preferably from 0.1 to 30% by weight, based on the total weight of the material.

Next, production examples of the purified culture liquid of Pleurotus sajorcaju, which is an active ingredient of the cosmetic material of the present invention, will be mentioned below.

METHODS OF MANUFACTURE

Production Example 1:

30 g of glucose, 2 g of yeast extracts, 2 g of peptone, 1 g of potassium dihydrogon phosphate, 0.5 g of magnesium sulfate, 0.1 f of sodium chloride, 0.1 g of calcuim chloride and 1,000 ml of pure water were dissolved under heat in a jar fermenter, sterilized in an autoclave at 121°C for 15 minutes and cooled to room temperature. Mycelia of Pleurotus sajorcaju were inoculated in this and cultivated by aerial shaking cultivation at 30°C for one week. After cultivation, the cultivated medium was subjected to centrifugation at 2,500 rpm to remove the mycelia therefrom, and it was then filtered through a filtration aid such as sellaite to obtain 500 g of a clear purified culture liquid of Pleurotus sajorcaju.

Production Example 2:

30 g of glucose, 2 g of yeast extract, 100 g of soybean milk. 2 g of peptone, 1 g of potassium dihydrogen phosphate, 0.5 g of magnesium sulfate, 0.1 g of sodium chloride, 0.1 g of calcium chloride and 800 ml of pure water were dissolved under heat in a jar fermenter, sterilized in an autoclave at 121°C for 15 minutes and cooled to room temperature. Mycelia of Pleurotus sajorcaju were inoculated in this and cultivated by aerial shaking cultivation, the cultivated medium was subjected to centrifugation at 2,000 rpm to remove the mycelia therefrom, and it was then filtered through a filtration aid such as sellaite to obtain 500 g of a clear purified culture liquid of Pleurotus sajorcaju.

Production Example 3:

50 mg of a protease (Actinase, product by Kaken Pharmaceutical Co.) was added to 1000 g of the purified culture liquid of Pleurotus sajorcaju as obtained in Production Example 2 and stirred for 18 hours at room temperature. The mixture was subjected to ultrafiltration with an ultrafilter for a molecular weight of 1,000 so that substances having a molecular weight of not less than 1,000 were removed. The resulting filtrate was distilled under reduced pressure to obtain 200 g of a purified culture medium of Pleurotus sajorcaju.

ANALYTICAL METHODS

Test results (data of demonstrated effects) of purified culture liquids to be used in the present invention and preparations containing them are measured using the following methods.

(1) Ultraviolet Damage Inhibiting Effect:

a) Test Method

$1 \times 10\grave{U}^5$ cells/0.1 ml of cultured mouse fibroblasts (3T3-LI Swiss Albino) were added to 4 ml of an Eagle's MEM medium containing 10 v/v % calf serum and incubated at 37°C in 5% $CO^2$ gaseous phase. On Day 3 after initiation of incubation, 30 MJ or 60 MJ ultraviolet rays (UVB) were irradiated thereto, and immediately after the ultraviolet irradiation, the medium was exchanged with a new one containing 1/5, 3/20, or 1/20 of 4 ml of the purified culture liquid of Pleurotus sajorcaju (as obtained in Production Example 3).

Two days after the ultraviolet irradiation, the cells were peeled with a mixes trypsin/EDTA solution and the number of the living cells was counted.

Apart from this, the purified culture liquid of Pleurotus sajorcaju having a determined concentration was added to the medium without ultraviolet irradiation and the cells were incubated in the same way. The number of living cells was also counted, and the survival percentage of the tested cells at the determined concentration of the purified culture liquid added was obtained.

Survival Percentage of Cells of Test Group (%)
= [(number of living cells under ultraviolet irradiation)/(number of living cells not under ultraviolet irradiation)] x 100.

Additionally, the survival percentage of control group cells was obtained in the same manner as above, except that the purified culture liquid of Pleurotus sajorcaju was not added.

Survival Percentage of Cells of Control Group (%)

= [(number of living cells under ultraviolet irradiation)/(number of living cells not under ultraviolet irradiation)] x 100.

b) Test Results:

As is shown in Table 1 below, the purified culture liquid of <u>Pleurotus sajorcaju</u> inhibits ultraviolet damage caused by 30 MJ or 60 MJ ultraviolet irradiation, as compared to the control group, when 1/5, 3/20, 1/10, or 1/20 of the liquid was added to the cells of the test group.

Table 1

| Ultravio let Irradiated Cells | Survival Percentage of Cells (%) | | | | |
|---|---|---|---|---|---|
| | Control Group | Test Group (with purified culture liquid of <u>Pleurotus sajorcaju</u> added) | | | |
| | | 1/20 | 1/10 | 3/20 | 1/5 |
| 30 MJ | 75 | 80 | 85 | 91 | 94 |
| 60 MJ | 63 | 68 | 76 | 85 | 90 |

(2) <u>B16 Cells Melanogenesis Inhibiting Effect:</u>

a) Test Method:

1/2, 1/4 and 1/8 of 10 ml of purified culture liquid of <u>Pleurotus sajorcaju</u> as obtained in anyone of the above-mentioned Production Examples were separately added to 10 mlof an Eagle's MEM medium containing 10 v/v % fetal bovine serum to give test groups A, B and C, respectively; the purified culture liquid was not added thereto for a control group.

$1.0 \times 10^5$ B16 cells were inoculated in each of the thus prepared test groups and control group, and then incubated therein at 37°C under 5 % $CO_2$ gaseous phase condition for 5 days. Exchange of the medium was carried out once during the incubation. After incubation, the cells were peeled and subjected to centrifugation (about 700 G), whereupon the black degree of the centrifuged pellets of the cells of each test group was visually compared with that of the control group.

b) Test Results:

The test results are shown below in Table 2.

Table 2

| Purified culture liquid of <u>Pleurotus sajorcaju</u> | Test Group A | Test Group B | Test Group C | Control Group |
|---|---|---|---|---|
| Production Example 1 | +++ | ++ | + | - |
| Production Example 2 | +++ | ++ | + | - |
| Production Example 3 | +++ | +++ | ++ | - |

(Criteria for Determination)
+ indicates the whiteness degree
+++ white
++ white to gray
+ gray
- black

(3) Anti-oxidating Effect

a) Test Method:

Test Samples
1) Pure water (control)
2) Vitamin E (30 pM)
3) Purified culture liquid of Pleurotus sajorcaju (obtained in Production Example 1) 0.8%
4) Purified culture liquid of Pleurotus sajorcaju (obtained in Production Example 2) 0.8%
5) Purified culture liquid of Pleurotus sajorcaju (obtained in Production Example 3) 0.8%
5.0 ml of any of the above-mentioned test samples was added to 10.0 ml of 1 M phosphate buffer of 1.0 ml of 500 mM linolic acid ethanol solution (pH 7.0) and 9.0 ml of ethanol, to prepar a sample solution. The respective sample solutions were kept in a dark place at 37°C for 9 days, whereupon the time-dependent variation of the peroxide value of each solution was measured by an iron rhodanide method. Precisely, 4.7 ml of 75% ethanol, 0.1 ml of 30% ammonium iron rhodanine (ammonium thiocyanate) and 0.1 ml of 3.5% hydrochloride solution of $2 \times 10\grave{U}^2$ M ferric chloride were added to 0.1 ml of each sample solution, and the absorbance at 500 nm a measured just after 3 minutes.

b) Test Results

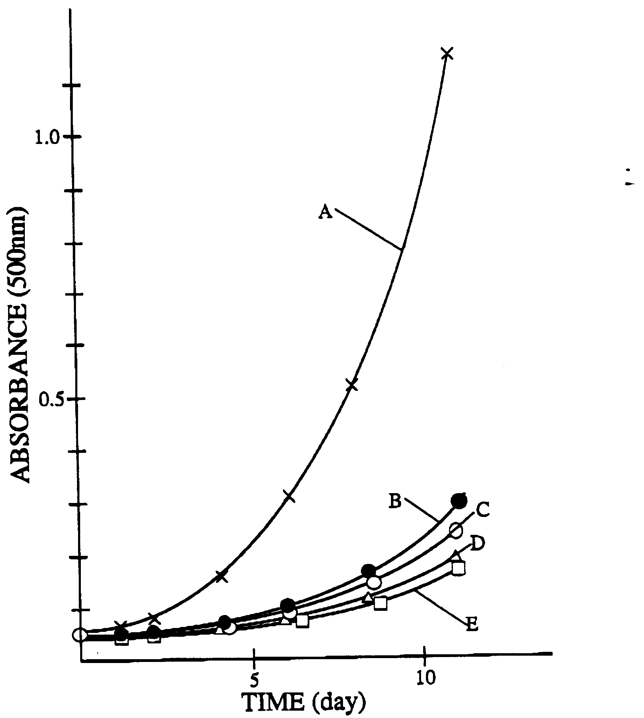

The test results obtained are shown in Figure 1 attached.

(4) Use Experiments:

(A) Effect for smoothing wrinkles:

a) Test Method:

The essence composition, according to Formulation Example 7, below was applied to the face skin of each of 20 female participants (ages 35 to 55) once every morning and evening (totaling twice a day) continuously for 3 months. After the continuous application test, the degree of anti-wrinkling effect of the sample was determined. Application of the sample to the face was effected by a half-face method where the essence sample of the present invention was applied to a half of the face while a control essence sample (not containing the purified culture liquid of Pleurotus sajorcaju of the invention) was applied to the other half of the face. Evaluation was effected by comparing the halves of the face with each other.

b) Test Results:

The smoothed degree of wrinkles was compared to the condition before application of the essence sample. As a result, a noticeable effect of smoothed wrinkles was admitted in the case of using the essence sample of the present invention. The results obtained are shown in Table 3 below.

Table 3

| Sample | Notice-ably smoothed | Fairly smoothed | Somewhat smoothed | Not Changed | Worsened | Total | Side Effect |
|---|---|---|---|---|---|---|---|
| Formula-tion 7 | 2 | 8 | 6 | 4 | 0 | 20 | 0 |
| Control | 0 | 1 | 4 | 15 | 0 | 20 | 0 |

(B) Anti-pigmentation Effect:

a) Test Method:

Samples used in the test:

5

Formulations 2 to 4 obtained in the examples mentioned below and a control emulsion as prepared in the same manner with exception of using pure water in place of the purified culture liquid were used as test samples.

1) Formulation 2 (Emulsion containing the purified culture liquid of Production Example 1).

2) Formulation 3 (Emulsion containing the purified culture liquid of Production Example 2)

3) Formulation 4 (Emulsion containing the purified culture liquid of Production Example 3)

4) Control (Emulsion containing pure water in place of the purified culture liquid of the invention).

Four sections each having a size of 1.5 cm X 1.5 cm were arranged in the inside of the upper arm of each of 50 participants, which were called section A, section B, section C and section D. Each section was irradiated with two FL20S.BLB lamps and two FL20S.E-30 lamps from a distance of 10 cm for 5 minutes.

Immediately after irradiation, formulation 2 was applied to section A; formulation 3 to section B; formulation 4 to section C; and control to section D, each three times a day for 7 days in every case. After 7 days, the degree of remedy of pigmentation of each section was determined.

b) Test Results:

The degree of remedy of pigmentation of the tested sections was evaluated by comparing the test group of sections A, B, and C with the control group of section D. The results obtained are shown in Table 4 below.

Table 4

| Section | Notice- ably remedied | Fairly remedied | Somewhat remedied | No Differ- ence | Worsened | Total | Side Effect |
|---|---|---|---|---|---|---|---|
| A | 9 | 28 | 7 | 6 | 0 | 50 | 0 |
| B | 8 | 26 | 8 | 8 | 0 | 50 | 0 |
| C | 9 | 25 | 8 | 8 | 0 | 50 | 0 |

As mentioned above, the cosmetic material of the present invention displayed an excellent anti-pigmentation effect.

(C) Effect of Preventing Formation of Dandruff and Urtication;

a) Test Method:

A tonic composition according to formulation Example 8 below, and a control tonic sample (not containing the purified culture liquid of _Pleurotus sajorcaju_ of the invention) were used. Ten male participants (ages 25 to 35) who were suffering from dandruff and urtication in the previous checkup were selected, and both samples were applied to them for the test.

The participants shampooed three times a week, and the shampooed hair was dried with a towel each times. After thus shampooing and drying, 4 ml of the control sample was rubbed into the scalp by sufficient massage each time. After three weeks, the degree of formation of dandruff and urtication of the scalp of each participant was evaluated with a four-rank evaluation by those skilled in the art, along with a physical consultation of the condition with each participant. Next, the tonic composition of Formulation Example 8 was applied to the same participants in the same manner as in the case of the control sample three times a week for three weeks. After the application, the degree of formation of dandruff and urtication of the scalp, if any, of each participant was evaluated in the same way.

The result was represented by the mean value of the ten participants.

(Criteria for degree of dandruff)

0 : Almost not dandruff formed.

1 : Dandruff seemingly formed.

2 : Dandruff positively formed.

3 : Extreme dandruff formed.

(Criteria for degree of urtication)
0 : The participants had almost no urtication.
1 : They had some urtication.
2 : They had positive urtication.
3 : They had extreme urtication.

b) Test Results:

As a result of determination of the degree of formation of dandruff of the participants, who tested both the tonic sample (formulation 8) and the control sample, and the degree of urtication of their scalp, the purified culture liquid of Pleurotus sajorcaju of the invention was obviously admitted to have an effect of preventing both formation of dandruff and urtication of the scalp. The test results obtained are shown in Table 5 below.

Table 5

|  | Degree of Dandruff | Degree of Urtication |
| --- | --- | --- |
| Tonic (Formulation Example 8) | 0.4 | 0.5 |
| Tonic (Control) | 2.4 | 2.1 |

The cosmetic material of the present invention is used in the form of a well-known preparation such as cream, emulsion, lotion, pack, hair tonic or the like. It is desirable for the purified culture liquid. Which is an active ingredient in the preparation, to be incorporated into the aqueous phase of the preparation. Some kinds of cosmetic materials would be composed of an aqueous phase and an oily phase in combination. In such cases, the active ingredient of the purified culture liquid of the invention should always be previously incorporated into the aqueous phase. The following are examples of producing preparations for external application:

Preparation Example 1: Cream

A moisturizing agent such as glycerin, sorbitol or the like is mixed with pure water to create an aqueous phase, and a purified culture liquid is incorporated thereinto.

Apart from this, an oily phase comprising solid oily components of bees wax, paraffin, microcrystalline wax, ceresine, higher fatty acids, hardened oil, etc.; liquid oily components of squaline, liquid paraffin, various ester oils, etc.; and other oily components of antiseptic surfactants, etc., is prepared.

Next, the aqueous phase is gradually heated. After it is heated to about 80°C, the oily phase that has also been heated to almost the same temperature is gradually added thereto and emulsified to give a cream.

Preparation Example 2: Emulsion

A moisturizing agent such as glycerin or the like, a pH adjusting agent such as an acid or alkali, and a purified culture liquid are added to pure water and mixwed, and ethanol is further added thereto to create an aqueous phase.

Apart from this, an oily phase is prepared by mixing solid oily components of bees wax, paraffin, etc.; liquid oily components of squalane, liquid paraffin, various ester oils, etc.; and other oily components of antiseptic surfactant, etc., under heat.

Next, the oily phase is added to the aqueous phase for pre-emulsification, and a protective colloid such as carboxymethyl cellulose or the like is added thereto and uniformly emulsified in a homomixer to create an emulsion.

Preparation Example 3: Lotion

A moisturizing agent such as glycerin, propylene glycol or the like, a skin nutrient, and a purified culture liquid are added to pure water. Apart from this, an alcohol phase containing an antiseptic and a perfume is prepared. The two are mixed at room temperature to make a lotion.

7

Preparation Example 4: Cream Pack

A moisturizing agent such as glycerin or the like, and a filiming agent such as polyvinyl alcohol, bee gum or the like are added to pure water along with a purified culture liquid, and swollen. If desired, a power of kaolin, talc, zinc oxide or the like is added thereto. Apart from this, an ethanol solution containing a perfume, an antiseptic, etc., is prepared. The latter is added to the former and kneaded to create a pasty cream pack.

Preparation Example 5: Hair Tonic

An aqueous phase is prepared by dissolving a moisturizing agent such as glycerin or the like, a chelating agent, and a purified culture liquid in pure water, and this is gradually added to ethanol little by little and uniformly mixed. The resulting mixture is filtered to make a hair tonic.

The following are concrete examples of formulations of the cosmetic material of the present invention. Such examples, however, do not whatsoever restrict the scope of the present invention.

Formulation Example 1: Cream

```
                                                          (wt. %)
           Polyoxyethylene Oleyl Ether (P.O.E.O.)          1.60
           Glycerin Monostearate                           3.00
           Bleached Bees Wax                                2.80
           Cetyl Alcohol*                                   3.00
           Stearic Acid                                     1.75
           Liquid Paraffin                                  7.10
           Butyl Methoxydibenzolmethane**                   0.50
           Squalane                                         4.50
           Glycerin                                         2.50
           Carbomer 940 ***                                 0.08
                         ****                               0.01
           Purified Culture Liquid as Produced in Production
           Example 3                                        0.10
           Pure Water containing small amounts of          73.06
           perfume and antiseptic.
           *     Cetanol, Available from _____
           **    Parsol 1789, Available from B.F. Goodrich Chemical Co.
           ***   Carbopol 940, Available from Givaudan Corp.
           ****  Clewat N, Available from _____
```

Formulation Example 2: Emulsion

|  | (wt. %) |
| --- | --- |
| Squalane | 5.00 |
| Petrolatum* | 2.00 |
| Bees Wax | 0.50 |
| Sorbitan Sesqui-oleate | 1.20 |
| Propylene Glycol | 5.00 |
| Ethanol | 5.00 |
| Carboxyvinyl Polymer (1% aqueous solution) | 20.00 |
| Potassium Hydroxide | 0.10 |
| Purified Culture Liquid as Produced in Production Example 1 | 10.00 |
| Pure Water containing small amounts of perfume, antiseptic and antioxidant | 51.20 |

*Vaseline, Available from Cheasebrough-Pond's, Inc.

Formulation Example 3: Emulsion

This is same as the emulsion of Formulation Example 2, except that a purified culture liquid as produced in Production Example 2 was used in place of the purified culture liquid as produced in Production Example 1.

Formulation Example 4: Emulsion

This is same as the emulsion of Formulation Example 2, except that a purified culture liquid as produced in Production Example 3 was used in place of the purified culture liquid as produced in Production Example 1.

Formulation Example 5: Lotion

|  | (wt. %) |
| --- | --- |
| Citric Acid | 0.40 |
| Calcium Carbonate | 0.20 |
| Ethanol | 5.00 |
| Propylene Glycol | 9.00 |
| Purified Culture Liquid as Produced in Production Example 1 | 30.00 |
| Pure Water | 55.40 |

In addition to these, small amounts of perfume and antiseptic were incorporated.

Formulation Example 6: Pack

|  | (wt. %) |
| --- | --- |
| Bee Gum | 5.00 |
| Squalane | 2.00 |
| Propylene Glycol | 5.00 |
| Zinc Oxide | 10.00 |
| Ethanol | 5.00 |
| Purified Culture Liquid as Produced in Production Example 2 | 2.00 |
| Pure Water | 71.00 |

In addition to these, small amounts of perfume and antiseptic were incorporated.

Formulation Example 7: Essence

|  | (wt. %) |
| --- | --- |
| Carboxyvinyl Polymer (1% aqueous solution) | 10.00 |
| Glycerin | 20.00 |
| Ethanol | 1.00 |
| Sodium Hyaluronate (0.1% aqueous solution) | 10.00 |
| Purified Culture Liquid as Produced in Production Example 2 | 10.00 |
| Pure Water | 49.00 |

In addition to these, small amounts of perfume and antiseptic were incorporated.

Formulation Example 8: Hair Tonic

|  | (wt. %) |
| --- | --- |
| Ethanol (95%) | 75.00 |
| Glycerin | 5.00 |
| Purified Culture Liquid as Produced in Production Example 2 | 5.00 |
| Pure Water | 15.00 |

In addition to these, small amounts of perfume and antiseptic were incorporated.
(Advantage of the Invention)
In accordance with the present invention, there is provided a cosmetic material containing the above-mentioned purified culture liquid of Pleurotus sajorcaju. When applied to the skin, the cosmetic material is quite free from any harmful side effects and it may effectively prevent aging of the skin as symptomized by wrinkles and pigmentation, such aging being promoted by ultraviolet rays. When the cosmetic material is applied to the scalp, it may effectively prevent formation of dandruff and urtication.
[Brief Explanation of the Drawing]
[Figure 1]
This shows test results of an antioxidating effect test.

**Claims**

1. A cosmetic material containing a purified liquid obtained from liquid cultivation of mycelis of <u>Pleurotus sajorcaju.</u>

2. A cosmetic composition according to claim 1 comprising from 0.1 to 30% by weight of said purified liquid and from 70 to 99.9% by weight of a dermatologically acceptable carrier, diluent or solvent.

3. A process for preparing a liquid useful in cosmetic applications which comprises aerobic cultivation of mycelia of <u>Pleurotus sajorcaju</u> in cultivation medium at a temperature in the range of 24 to 35 C and thereafter separating the liquid portion from the rest of the fermentation broth.

4. A process according to Claim 3 wherein said liquid is subsequently treated with a protease and subjected to ultrafiltration to remove substances having a molecular weight of over 1000.

European Patent Office

**EUROPEAN SEARCH REPORT**

Application Number

| DOCUMENTS CONSIDERED TO BE RELEVANT | | | EP 92301068.0 |
|---|---|---|---|
| Category | Citation of document with indication, where appropriate, of relevant passages | Relevant to claim | CLASSIFICATION OF THE APPLICATION (Int. Cl.5) |
| A | PATENT ABSTRACTS OF JAPAN, unexamined applications, C field, vol. 10, no. 287, September 30, 1986, THE PATENT OFFICE JAPANESE GOVERNMENT page 10 C 375 * Kokai-no. 61-103 815 (HITOSHI NAGAOKA) * | 1-4 | A 61 K 7/00 A 61 K 7/42 A 61 K 7/48 A 61 K 7/06 |
| A | PATENT ABSTRACTS OF JAPAN, unexamined applications, C field, vol. 10, no. 287, September 30, 1986, THE PATENT OFFICE JAPANESE GOVERNMENT page 10 C 375 * Kokai-no. 61-103 816 (HITOSHI NAGAOKA) * | 1-4 | |
| A | PATENT ABSTRACTS OF JAPAN, unexamined applications, C field, vol. 12, no. 482, December 15, 1988, THE PATENT OFFICE JAPANESE GOVERNMENT page 43 C 553 * Kokai-no. 63-198 608 (YUSUKE SHIGYO) * | 1-4 | TECHNICAL FIELDS SEARCHED (Int. Cl.5)  A 61 K 7/00 |
| A | EP - A - 0 111 631 (SIDHU, T.S.) * Claims * | 1,2 | |

The present search report has been drawn up for all claims

| Place of search | Date of completion of the search | Examiner |
|---|---|---|
| VIENNA | 29-04-1992 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P0401)